# EUROPEAN PATENT APPLICATION

(11) **EP 4 071 242 A1**
(43) Date of publication of application: **12.10.2022**
(21) Application number: 21167053.4
(22) Date of filing: 06.04.2021
(51) Int. Cl.: C12N 9/26, C12P 19/14, A21D 8/04, A23L 33/18, A23L 29/30, C12N 9/28

(54) **AMYLASE POLYPEPTIDES WITH IMPROVED PROPERTIES**

(71) Applicant: DuPont Nutrition Biosciences ApS, 1411 Køpenhaven K (DK)
(72) Inventor: DOIG, Steven, DK-1411 Copenhagen K (DK); FRISCH, Ryan, DK-1411 Copenhagen K (DK); HAANING, Svend, DK-1411 Copenhagen K (DK); KOOPMAN, Frank, DK-1411 Copenhagen K (DK); KRAGH, Karsten Matthias, DK-1411 Copenhagen K (DK); KRAGH, Lene, DK-1411 Copenhagen K (DK); LEEFLANG, Chris, DK-1411 Copenhagen K (DK); PRICELIUS, Sina, DK-1411 Copenhagen K (DK)
(74) Representative: D Young & Co LLP

(57) **Abstract**

This invention relates to polypeptides, more specifically amylase polypeptides and nucleic acids encoding these, and their uses e.g. as non-maltogenic exoamylases in producing food or feed products.

## Description

### FIELD OF THE INVENTION

This invention relates to polypeptides, specifically amylase polypeptides and nucleic acids encoding these, and their uses e.g. as non-maltogenic exoamylases in producing food or feed products.

### BACKGROUND TO THE INVENTION

Amylases can ameliorate problems inherent in conversion of cereal starches such as in baking. A bread crumb can be characterized by the molecular networks present therein. Bread staling is a dynamic process where changes occur in the molecular networks in the bread crumb. When bread stales, bread loses crumb softness and crumb moisture with a concomitant loss of bread resilience. As a result, crumbs become less elastic, and the bread develops a leathery crust.

Without being bound by any particular theory it is believed that two molecular networks are particularly important for the textural properties of bread such as bread softness and bread resilience; 1) the network formed by gluten proteins and 2) the network formed by the starch polymer amylopectin. Amylopectin is a very large, branched molecule consisting of chains of α-D-glucopyranosyl units joined by (1-4) linkages, where the chains are attached by α-(1-6) linkages to form branches (also called side-chains). The properties of both gluten and amylopectin networks can be affected by starch modifying amylase enzymes even though the effect on the gluten network is indirect. In the dough most of the amylopectin is found inside the starch granules in a crystalline form. During baking the starch granules will take up water in a process called gelatinization and some of the amylopectin will leak out of the starch granule. In the freshly baked bread, most of the amylopectin will no longer be in the crystalline form but rather form an amorphous network throughout the bread. Over time the amylopectin will recrystallize, a process called retrogradation, thereby forming a stronger network which in turn lead to the decrease in bread softness and resilience associated with bread staling. The speed and extent of amylopectin retrogradation is dependent on the length of amylopectin sidechains with shorter sidechains slowing the retrogradation.

As amylopectin recrystallizes it binds water which lead to water migration within the bread crumb. An important consequence of this water migration is that the gluten network loses some of the plasticizing water and become less flexible. This is associated with a loss of bread resilience.

To slow the staling of bread products it is very common in the bread making industry to use so called anti-staling amylases. An effective anti-staling amylase needs to be highly resistant to temperature inactivation in order to remain active throughout the bake. Also, it needs to be effective at shortening the amylopectin sidechains to retard amylopectin recrystallization. Most amylases used for anti-staling are predominantly exo-acting meaning that they mainly hydrolyze amylopectin at bonds near the ends of the side chains, thereby shortening the side-chains but having little impact on the size of the amylopectin. However, most anti-staling amylases also have some limited endo activity - meaning that they are able to hydrolyze bonds internally in the amylopectin backbone which have a much greater impact on the size of the amylopectin.

It is believed that some limited endo activity is beneficial in terms of bread softness, volume and crumb moisture but too much endo activity can negatively affect the quality of the final bread product by producing a sticky or gummy crumb. Without being bound by any particular theory it is believed that an effective anti-staling amylase needs to have both exo- and endo activity. Some of the most successful anti-staling amylases in the market are variants of a maltotetraose-forming amylase (G4amylase) from *Pseudomonas saccharophila.*

However, there is a trend in the market towards longer shelf-live where the breads are expected to maintain a high level of softness and resilience even after 21 days of storage. To enable longer shelf-live there is a need in the market for even better anti-staling G4 amylases providing higher resilience and softness at extended storage times.

### SUMMARY OF THE INVENTION

In accordance with an aspect of the present invention, a *Pseudomonas saccharophila* amylase (PS4) variant or an amylase active fragment thereof is presented, wherein the variant has a sequence having at least 70% sequence identity to the amino acid sequence of SEQ ID NO: 1, SEQ ID NO:18, SEQ ID NO:19, SEQ ID NO:20, SEQ ID NO:21, SEQ ID NO:23, or SEQ ID NO:22, and wherein the PS4 variant or an amylase active fragment thereof has an amino acid substitution at one or more of the following positions 31, 58, 172, 230, 362 118, 209, 249, 301, 369, 377, 404, 407 and 421.

According to any aspect of the present invention, the degree of identity with regard to an amino acid sequence may be determined over residues 1 to 429 of SEQ ID NO: 1 to SEQ ID NO: 23, such as SEQ ID NO: 1, SEQ ID NO:18, SEQ ID NO:19, SEQ ID NO:20, SEQ ID NO:21, SEQ ID NO:23, or SEQ ID NO:22.

Optionally, the PS4 variant or an amylase active fragment thereof has at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence of SEQ ID NO: 1, SEQ ID NO:18, SEQ ID NO:19, SEQ ID NO:20, SEQ ID NO:21, SEQ ID NO:23 or SEQ ID NO:22.

Optionally, the PS4 variant or an amylase active fragment thereof has one or more amino acid substitution corresponding to 31V, 58P, 172R, 230S, 362P, 118E, 209N, 249S, 301P, 369F, 377Q, 404L, 407F or 421S.

Optionally, the PS4 variant or an amylase active fragment thereof has one or more of the following amino acid substitutions 68S, 145Q, 155P, 220M, 3V, 1101, 169H, 179V, 188E, 188R, 205Q, 313A, 313W, 367T, 399E, 422Y, 1101, 179N, 188E or 313W.

Optionally, the PS4 variant or an amylase active fragment thereof has one or more of the following substitutions 34Q, 145D, 179R, 200G, 223W, 169R, 179N or 420G.

Optionally, the PS4 variant or an amylase active fragment thereof has an amino acid sequence that has at least 70% sequence identity with SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16 or SEQ ID NO:17.

Optionally, the PS4 variant or an amylase active fragment thereof has an amino acid sequence that has at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, and 99% sequence identity with SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16 or SEQ ID NO:17.

Optionally, the PS4 variant or an amylase active fragment thereof has the amino acid sequence of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16 or SEQ ID NO:17.

Optionally, the PS4 variant or an amylase active fragment thereof has a melting temperature of at least about 0.5°C, 1, 2°C, 3°C, 4°C, 5°C, 6°C or 7°C greater than the melting temperature of SEQ ID NO:18.

Optionally, the PS4 variant or an amylase active fragment thereof has an exo to endo activity ratio at least about 50%, 75%, 100%, 200%, 300%, 400%, 500%, 600%, 700% or 800% of the exo to endo activity ratio of SEQ ID NO:18.

In another aspect of the present invention, the use of a PS4 variant or an amylase active fragment thereof as set forth above as a food or feed additive is presented.

In another aspect of the present invention, a process is presented for treating starch by contacting starch with a PS4 variant or an amylase active fragment thereof as described above and allowing the PS4 variant or an amylase active fragment thereof to generate from the starch one or more linear products.

In another aspect of the present invention, the use of a PS4 variant or an amylase active fragment thereof as described above in preparing a food or feed product is presented.

In another aspect of the present invention, a process for preparing a food or feed product is presented having the step of admixing a PS4 variant or an amylase active fragment thereof as described above with a food or feed ingredient.

Optionally, the food product in the use or process is a dough or a dough product. More preferably, the dough product is a processed dough product.

Optionally, the food product in the use or process is a bakery product.

According to another aspect of the present invention, a process for making a bakery product is presented having the steps of (a) providing a starch medium; (b) adding to the starch medium a PS4 variant or an amylase active fragment thereof as described above; and (c) applying heat to the starch medium during or after step (b) to produce a bakery product.

In another aspect of the present invention a food product, feed product, dough product or a bakery product obtained by a use or process as described above is presented. According to another aspect of the present invention, a method of making a saccharide syrup is presented having the step of adding a PS4 variant or an amylase active fragment thereof as described above to a granular starch liquefact to form a saccharide syrup.

In another aspect of the present invention, a dough is presented comprising a PS4 variant or an amylase active fragment thereof as set forth above. Optionally, the dough has at least one additional enzyme useful for improving dough and/or a baked product made therefrom. Optionally, the additional enzyme is one or more of an amylase wherein the amylase is different than the PS4 variant or an amylase active fragment thereof as set forth above, cyclodextrin glucanotransferase, peptidase, transglutaminase, lipase, galactolipase, phospholipase, cellulase, hemicellulase, protease, protein disulfide isomerase, glycosyltransferase, peroxidase, lipoxygenase, laccase, and oxidase. Optionally, the additional enzyme is an amylase. Optionally, the amylase is an exoamylase. Optionally, the exoamylase is a maltogenic amylase. Optionally, the maltogenic amylase is a polypeptide according to SEQ ID NO:24. Optionally, the exoamylase is a non-maltogenic amylase. Optionally, the additional enzyme is a phospholipase or a galactolipase.

In another aspect, a method is presented of preparing a baked product having the step of baking a dough as described above. Another aspect is such a baked product.

In another aspect of the present invention, a method is presented having the step of admixing a dough component selected from the group consisting of flour, salt, water, sugar, fat, lecithin, oil and yeast with a PS4 variant as described above. Optionally, the method has the additional step of adding at least one additional enzyme useful for improving dough and/or a baked product made therefrom. Optionally, the additional enzyme is one or more of an amylase wherein the amylase is different than the PS4 variant or an amylase active fragment thereof as set forth above, cyclodextrin glucanotransferase, peptidase, transglutaminase, lipase, galactolipase, phospholipase, cellulase, hemicellulase, protease, protein disulfide isomerase, glycosyltransferase, peroxidase, lipoxygenase, laccase, and oxidase. Optionally, the additional enzyme is an amylase. Optionally, the amylase is an exoamylase. Optionally, the exoamylase is a maltogenic amylase. Optionally, the maltogenic amylase is a polypeptide according to SEQ ID NO:24. Optionally, the exoamylase is a non-maltogenic amylase. Optionally, the additional enzyme is a phospholipase or a galactolipase.

In another aspect a nucleic acid is presented which is capable of encoding a PS4 variant or an amylase active fragment thereof as described above. In other words, a nucleic acid is presented which encodes a PS4 variant or an amylase active fragment thereof as described above. Another aspect is an expression vector having or comprising the nucleic acid. Yet another aspect is a host cell having or comprising the expression vector. Optionally, the host cell is a bacterial, fungal or yeast cell.

### BRIEF DESCRIPTION OF THE FIGURES

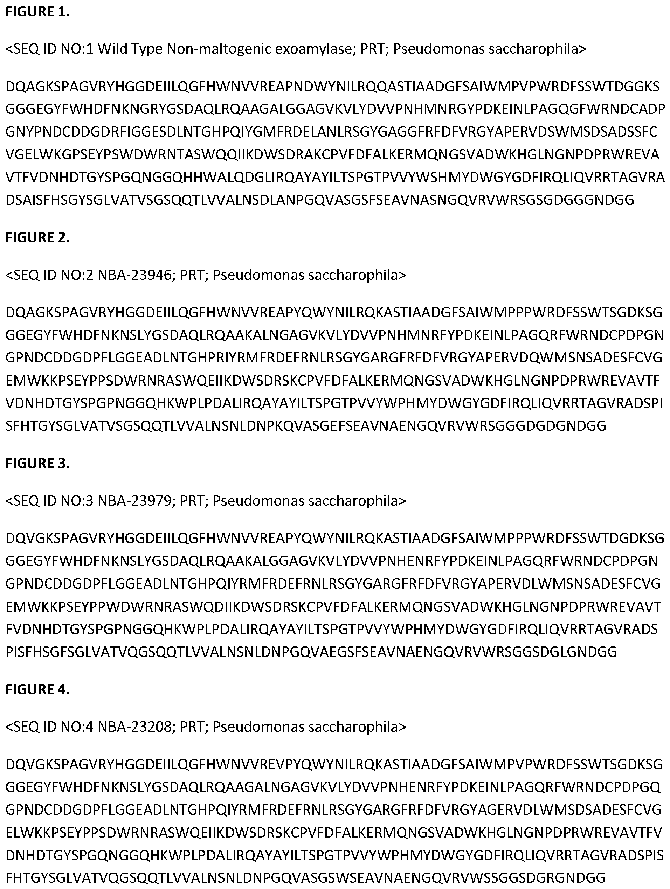

Figure 1: SEQ ID NO:1 sets forth the mature protein sequence of a non-maltogenic exoamylase from *Pseudomonas saccharophila.*
Figure 2: SEQ ID NO:2 sets forth the mature protein sequence of non-maltogenic exoamylase variant NBA-23946.
Figure 3: SEQ ID NO:3 sets forth the mature protein sequence of non-maltogenic exoamylase variant NBA-23979.
Figure 4: SEQ ID NO:4 sets forth the mature protein sequence of non-maltogenic exoamylase variant NBA-23208.

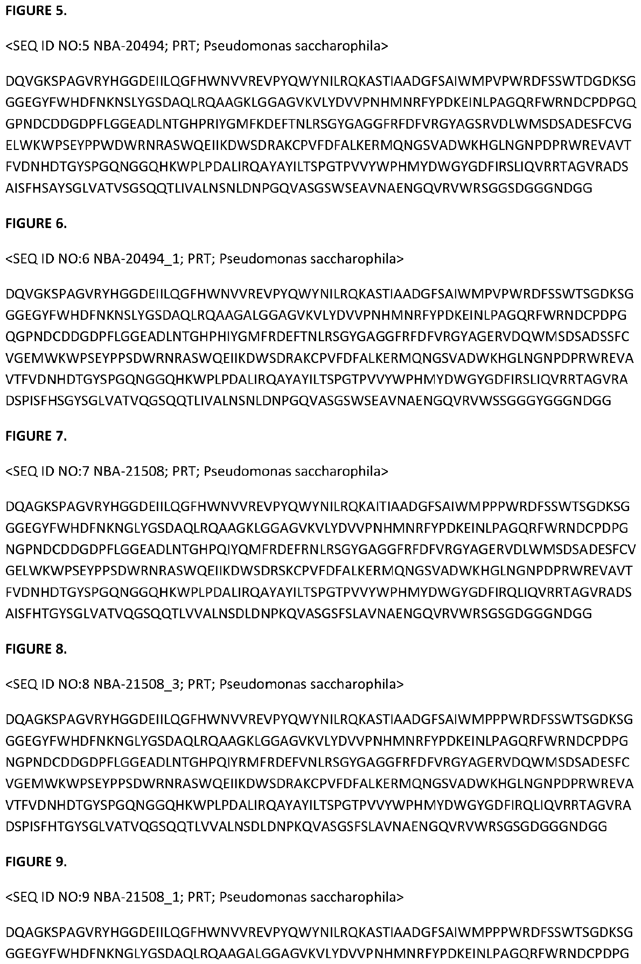

Figure 5: SEQ ID NO:5 sets forth the mature protein sequence of non-maltogenic exoamylase variant NBA-20494.
Figure 6: SEQ ID NO:6 sets forth the mature protein sequence of non-maltogenic exoamylase variant NBA-20494_1.
Figure 7: SEQ ID NO:7 sets forth the mature protein sequence of non-maltogenic exoamylase variant NBA-21508.
Figure 8: SEQ ID NO:8 sets forth the mature protein sequence of non-maltogenic exoamylase variant NBA-21508_3.
Figure 9: SEQ ID NO:9 sets forth the mature protein sequence of non-maltogenic exoamylase variant NBA-21508_1.

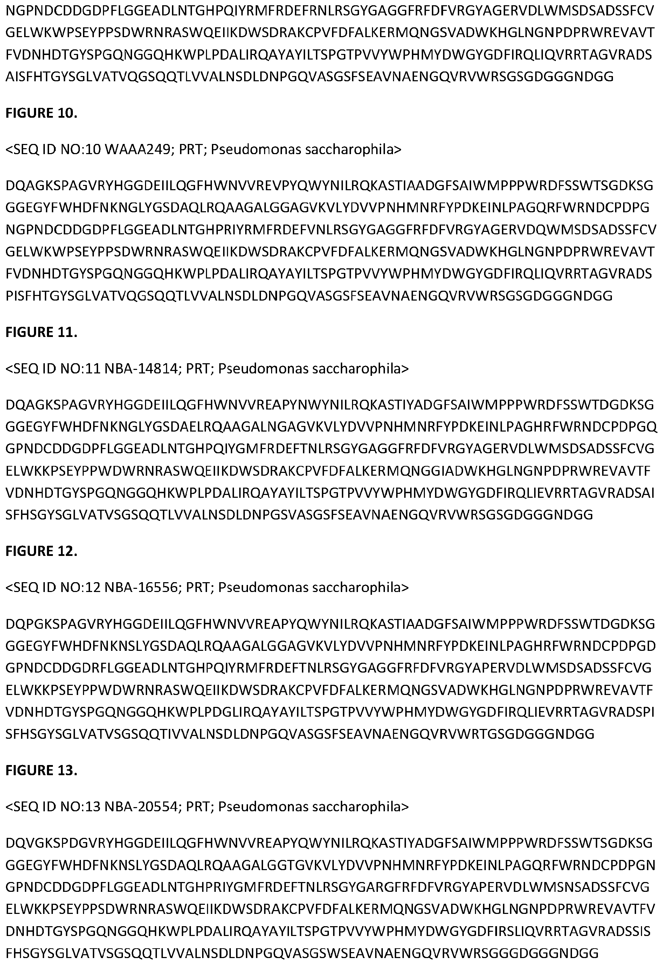

Figure 10: SEQ ID NO:10 sets forth the mature protein sequence of non-maltogenic exoamylase variant WAAA249.
Figure 11: SEQ ID NO:11 sets forth the mature protein sequence of non-maltogenic exoamylase variant
   NBA-14814.
Figure 12: SEQ ID NO:12 sets forth the mature protein sequence of non-maltogenic exoamylase variant
   NBA-16556.
Figure 13: SEQ ID NO:13 sets forth the mature protein sequence of non-maltogenic exoamylase variant
   NBA-20554.

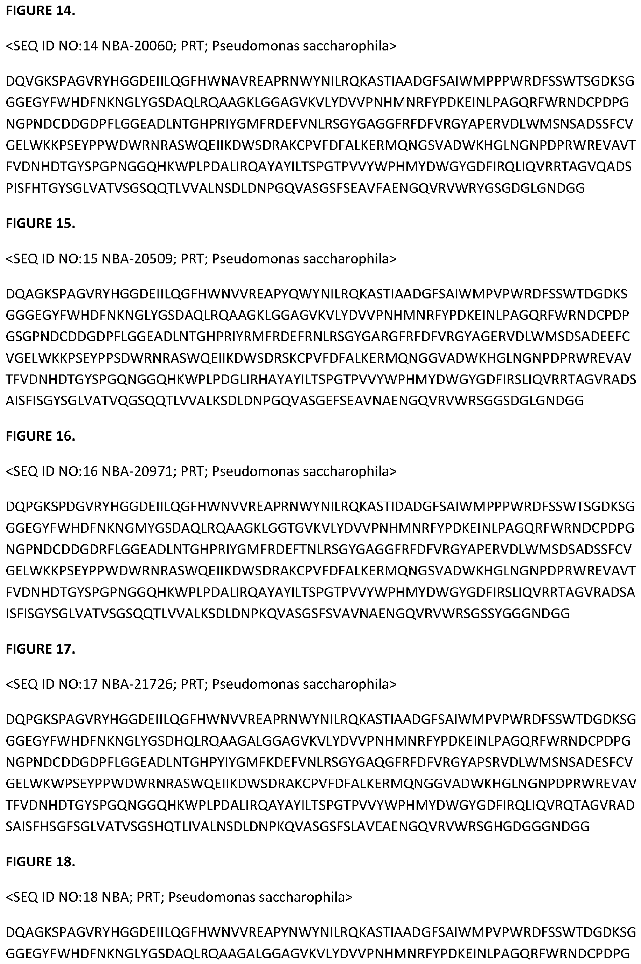

Figure 14: SEQ ID NO:14 sets forth the mature protein sequence of non-maltogenic exoamylase variant
   NBA-20060.
Figure 15: SEQ ID NO:15 sets forth the mature protein sequence of non-maltogenic exoamylase variant
   NBA-20509.
Figure 16: SEQ ID NO:16 sets forth the mature protein sequence of non-maltogenic exoamylase variant
   NBA-20971.
Figure 17: SEQ ID NO:17 sets forth the mature protein sequence of non-maltogenic exoamylase variant
   NBA-21726.
Figure 18: SEQ ID NO:18 sets forth the mature protein sequence of non-maltogenic exoamylase variant
   NBA.

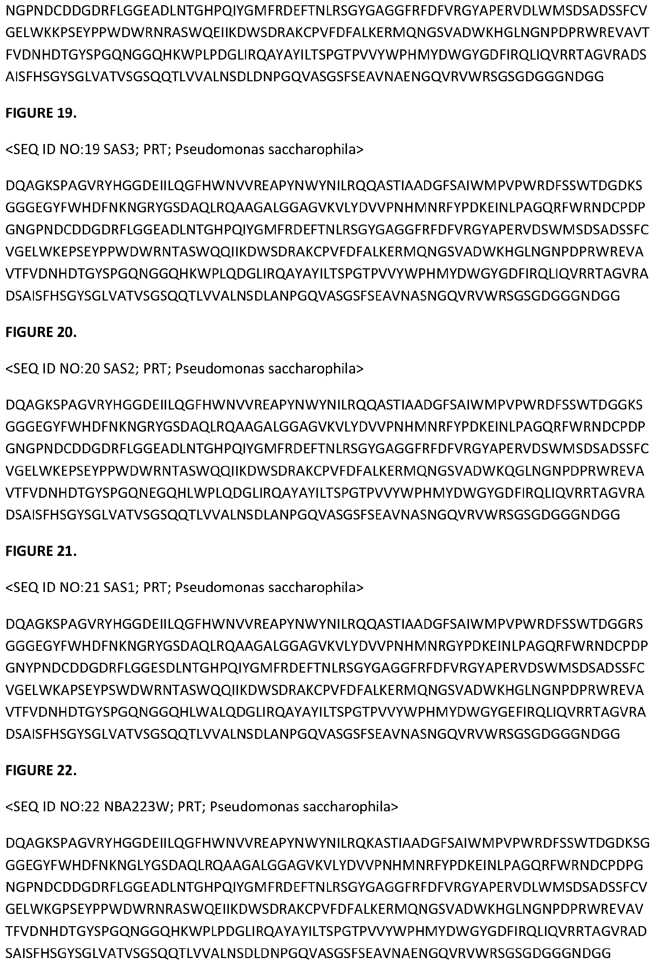

Figure 19: SEQ ID NO:19 sets forth the mature protein sequence of non-maltogenic exoamylase variant
   SAS3.
Figure 20: SEQ ID NO:20 sets forth the mature protein sequence of non-maltogenic exoamylase variant
   SAS2.
Figure 21: SEQ ID NO:21 sets forth the mature protein sequence of non-maltogenic exoamylase variant
   SAS1.
Figure 22: SEQ ID NO:22 sets forth the mature protein sequence of non-maltogenic exoamylase variant
   NBA223G.

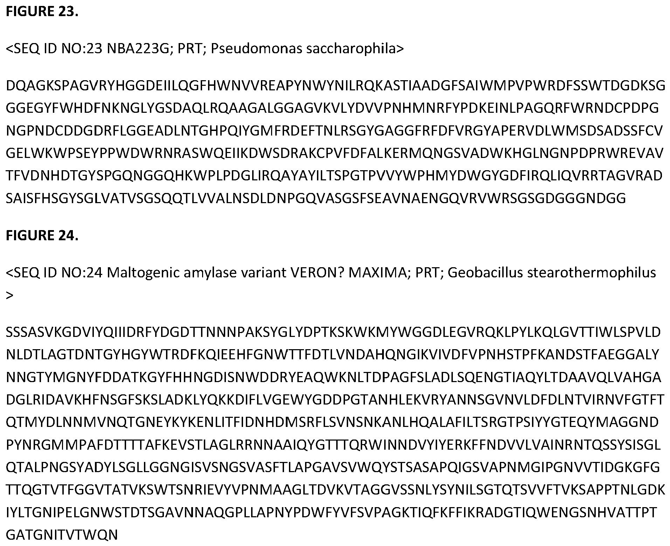

Figure 23: SEQ ID NO:23 sets forth the mature protein sequence of non-maltogenic exoamylase variant
   NBA223W.
Figure 24: SEQ ID NO:24 sets forth the mature protein sequence of a maltogenic amylase variant.

### DETAILED DESCRIPTION OF THE INVENTION

### General definitions

Unless defined otherwise, all technical and scientific terms used herein have the same meaning as commonly understood by one of ordinary skill in the art to which this disclosure belongs. This disclosure is not limited by the exemplary methods and materials disclosed herein, and any methods and materials similar or equivalent to those described herein can be used in the practice or testing of embodiments of this disclosure. Numeric ranges are inclusive of the numbers defining the range.

The headings provided herein are not limitations of the various aspects or embodiments of this disclosure which can be had by reference to the specification as a whole. Accordingly, the terms defined immediately below are more fully defined by reference to the specification as a whole.

As used herein, the term "polynucleotide" it is synonymous with the term "nucleotide sequence" and/or the term "nucleic acid sequence". Unless otherwise indicated, any nucleic acid sequences are written left to right in 5' to 3' orientation.

The term "protein", as used herein, includes proteins, polypeptides, and peptides. As used herein, the term "amino acid sequence" is synonymous with the term "polypeptide" and/or the term "protein". In the present disclosure and claims, the name of the amino acid, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code. Unless otherwise indicated, any amino acid sequences are written left to right in amino to carboxy orientation.

Other definitions of terms may appear throughout the specification. Before the exemplary embodiments are described in more detail, it is to understand that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The terms "comprising", "comprises", "has", "having" and "comprised of' as used herein are synonymous with "including", "includes" or "containing", "contains", and are inclusive or open-ended and do not exclude additional, non-recited members, elements or method steps. The terms "comprising", "comprises", "has", "having" and "comprised of' also include the term "consisting of.

The publications discussed herein are provided solely for their disclosure prior to the filing date of the present application. Nothing herein is to be construed as an admission that such publications constitute prior art to the claims appended hereto.

### Sequence identity

In addition to the specific amino acid sequences and polynucleotides mentioned herein, the present invention encompasses variants, homologues, derivatives and fragments thereof.

The term "variant" is used to mean a nucleotide sequence or amino acid sequence which differs from a wild-type sequence.

For example, a variant may include substitutions, insertions, deletions, truncations, transversions and/or inversions at one or more position(s) relative to a wild-type sequence. Variants can be made using methods known in the art for example site scanning mutagenesis, insertional mutagenesis, random mutagenesis, site-directed mutagenesis and directed-evolution as well as using recombinant methods well known in the art. Polynucleotide sequences encoding variant amino acid sequences may readily be synthesized using methods known in the art.

In some aspects, the variant is a naturally occurring nucleotide sequence or amino acid sequence which differs from a wild-type sequence. For example, the variant may be a natural genetic variant.

In one aspect, the PS4 variant or an amylase active fragment thereof is an isolated PS4 variant or isolated amylase active fragment thereof.

The term "isolated" means that the sequence is at least substantially free from at least one other component with which the sequence is naturally associated in nature and as found in nature.

In one aspect, "isolated variant" or isolated amylase active fragment thereof as used herein refers to a polypeptide which is at least 30% pure, at least 40% pure, at least 60% pure, at least 80% pure, at least 90% pure, and at least 95% pure, as determined by SDS-PAGE.

In some aspects, the variant is an engineered variant. For example, the variant may be engineered by recombinant methods.

In one embodiment, a "wild-type sequence" is SEQ ID NO: 1, SEQ ID NO:18, SEQ ID NO: 19, SEQ ID N0:20, SEQ ID NO:21, SEQ ID NO:23, or SEQ ID NO:22.

The term "homologue" means an entity having a certain homology with the subject nucleotide sequences. Here, the term "homology" can be equated with "identity".
In the present context, a homologous sequence is taken to include an amino acid or a nucleotide sequence which may be at least 70, 75, 80, 85 or 90% identical, preferably at least 95%, 96%, 97%, 98 % or 99% identical to the subject sequence. Typically, the homologues will comprise the same active sites etc. as the subject amino acid sequence for instance. Although homology can also be considered in terms of similarity (i.e. amino acid residues having similar chemical properties/functions), in the context of the present invention it is preferred to express homology in terms of sequence identity.

Suitably, a homologue of a PS4 variant according to the present invention is an active amylase. In other words, the homologue of a PS4 variant according to the present invention has amylase activity.

In one aspect, a homologous sequence is taken to include an amino acid sequence or nucleotide sequence which has one or several additions, deletions and/or substitutions compared with the subject sequence. Homology comparisons can be conducted by eye, or more usually, with the aid of readily available sequence comparison programs. These commercially available computer programs can calculate % homology between two or more sequences.

Percent homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons.

Calculation of maximum % homology or % identity therefore firstly requires the production of an optimal alignment, taking into consideration gap penalties.

The relationships between protein sequences are usually discovered by aligning them together and assigning this alignment a score. There are two main types of sequence alignment. Pairwise sequence alignment only compares two sequences at a time and multiple sequence alignment compares many sequences. Two important algorithms for aligning pairs of sequences are the Needleman-Wunsch algorithm and the Smith-Waterman algorithm. Popular tools for sequence alignment include BLAST for pairwise alignment, and ClustalW, MUSCLE, or MAFFT for multiple alignment.

There are two types of pairwise alignments: local and global alignments. A local alignment is an alignment of two sub-regions of a pair of sequences. This type of alignment is appropriate when aligning two sequences that may have local regions of similarity embedded in a background of a non-homologous sequence. For local alignments, the Smith-Waterman algorithm is the most commonly used. Other pairwise alignment tools include BLAST. A global alignment is a sequence alignment over the entire length of two or more protein sequences. In a global alignment, the sequences are assumed to be homologous along their entire length. An efficient algorithm for global alignment was described by Needleman and Wunsch 1970. Multiple protein sequence alignment tools such as CLUSTALW, MAFFT and MUSCLE are applied to evaluate similarities across multiple protein sequences.

A suitable computer program for carrying out such an alignment is available in the Vector NTI (Invitrogen Corp.) software package, which uses an algorithm comparable to the CLUSTAL W iterative multiple sequence alignment algorithm (Higgins DG & Sharp PM (1988), Gene 73(1), 237-244). Examples of software that can perform sequence comparisons include, but are not limited to, the BLAST package (see Ausubel et al., 1999, Short Protocols in Molecular Biology, 4th Ed - Chapter 18*),* BLAST 2 (see FEMS Microbiol Lett, 1999, 174(2): 247-50; FEMS Microbiol Lett, 1999, 177(1): 187-8), FASTA (Altschul et al., 1990, J. Mol. Biol., 403-410) and AlignX for example. At least BLAST, BLAST 2 and FASTA are available for offline and online searching (see Ausubel *et al.,* 1999, pages 7-58 to 7-60).

Although the % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Once the software has produced an optimal alignment, it is possible to calculate % homology, preferably % sequence identity. The software typically does this as part of the sequence comparison and generates a numerical result.

Gap Penalties may be used when determining a sequence identity. Examples of parameters used for a pairwise alignment are as set forth in Tables 1 and 2 below.

**Table 1.**

| FOR BLAST | |
|---|---|
| GAP OPEN | 9 |
| GAP EXTENSION | 2 |

**Table 2.**

| FOR CLUSTAL | DNA | PROTEIN |
|---|---|---|
| Weight Matrix | IUB | Gonnet 250 |
| GAP OPENING | 15 | 10 |
| GAP EXTEND | 6.66 | 0.1 |

In one embodiment, CLUSTAL may be used with the gap penalty and gap extension set as defined above.

Suitably, the degree of identity with regard to a nucleotide sequence is determined over at least 20 contiguous nucleotides, preferably over at least 30 contiguous nucleotides, preferably over at least 40 contiguous nucleotides, preferably over at least 50 contiguous nucleotides, preferably over at least 60 contiguous nucleotides, preferably over at least 100 contiguous nucleotides.

Suitably, the degree of identity with regard to a nucleotide sequence is determined over at least 100 contiguous nucleotides, preferably over at least 200 contiguous nucleotides, preferably over at least 300 contiguous nucleotides, preferably over at least 400 contiguous nucleotides, preferably over at least 500 contiguous nucleotides, preferably over at least 600 contiguous nucleotides, preferably over at least 700 contiguous nucleotides, preferably over at least 800 contiguous nucleotides.

Suitably, the degree of identity with regard to a protein (amino acid) sequence is determined over at least 100 contiguous amino acids, preferably over at least 200 contiguous amino acids, preferably over at least 300 contiguous amino acids.

For example, the degree of identity with regard to the amino acid sequence of a fragment may be determined over the length of the fragment sequence. In other words, the degree of identity with regard to the amino acid sequence of a fragment may be determined over the length of the shorter sequence.

Preferably, the degree of identity with regard to an amino acid or protein sequence may be determined over the whole mature sequence taught herein, such as over SEQ ID NO:1 or SEQ ID NO:18 disclosed herein.

In one embodiment, the degree of identity with regard to an amino acid or protein sequence may be determined over residues 1 to 429 of SEQ ID NO: 1 to SEQ ID NO:23. In one embodiment, the degree of identity with regard to an amino acid or protein sequence may be determined over residues 1 to 429 of SEQ ID NO: 1, SEQ ID NO:18, SEQ ID NO:19, SEQ ID NO:20, SEQ ID NO:21, SEQ ID NO:23, or SEQ ID NO:22.

In the present context, the term "query sequence" means a homologous sequence or a foreign sequence, which is aligned with a subject sequence in order to see if it falls within the scope of the present invention. Accordingly, such query sequence can for example be a prior art sequence or a third party sequence.

In one preferred embodiment, the sequences are aligned by a global alignment program and the sequence identity is calculated by identifying the number of exact matches identified by the program divided by the length of the subject sequence.

In one embodiment, the degree of sequence identity between a query sequence and a subject sequence is determined by 1) aligning the two sequences by any suitable alignment program using the default scoring matrix and default gap penalty, 2) identifying the number of exact matches, where an exact match is where the alignment program has identified an identical amino acid or nucleotide in the two aligned sequences on a given position in the alignment and 3) dividing the number of exact matches with the length of the subject sequence.

In yet a further preferred embodiment, the global alignment program is selected from the group consisting of CLUSTAL and BLAST (preferably BLAST) and the sequence identity is calculated by identifying the number of exact matches identified by the program divided by the length of the subject sequence.

The sequences, such as PS4 variant sequences or amylase active fragments thereof, may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other as set forth in Table 3.

**Table 3.**

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by synthetic amino acids (e.g. unnatural amino acids) include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, B-alanine*, L-a-amino butyric acid*, L-g-amino butyric acid*, L-a-amino isobutyric acid*, L-e-amino caproic acid^{#}, 7-amino heptanoic acid*, L-methionine sulfone^{#}*, L-norleucine*, L-norvaline*, p-nitro-L-phenylalanine*, L-hydroxyproline^{#}, L-thioproline*, methyl derivatives of phenylalanine (Phe) such as 4-methyl-Phe*, pentamethyl-Phe*, L-Phe (4-amino)^{#}, L-Tyr (methyl)*, L-Phe (4-isopropyl)*, L-Tic (1,2,3,4-tetrahydroisoquinoline-3-carboxyl acid)*, L-diaminopropionic acid^{#} and L-Phe (4-benzyl)*.

The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or b-alanine residues. A further form of variation, involves the presence of one or more amino acid residues in peptoid form, will be well understood by those skilled in the art. For the avoidance of doubt, "the peptoid form" is used to refer to variant amino acid residues wherein the a-carbon substituent group is on the residue's nitrogen atom rather than the a-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The nucleotide sequences for use in the present invention may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences of the present invention.

The present invention also encompasses the use of nucleotide sequences that are complementary to the sequences presented herein.

Other variants of the sequences described herein may be obtained for example by probing DNA libraries made from a range of individuals, for example individuals from different populations. In addition, other homologues may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

### General recombinant DNA methodology techniques

The present invention employs, unless otherwise indicated, conventional techniques of biochemistry, molecular biology, microbiology and recombinant DNA, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N. Y.); B. Roe, J. Crabtree, and A. Kahn, 1996, DNA Isolation and Sequencing: Essential Techniques, John Wiley & Sons; M. J. Gait (Editor), 1984, Oligonucleotide Synthesis: A Practical Approach, IrI Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

### Detailed Description of the Preferred Embodiments

In accordance with an aspect of the present invention, a *Pseudomonas saccharophila* amylase (PS4) variant or an amylase active fragment thereof is presented, wherein the variant has a sequence having at least 70% sequence identity to the amino acid sequence of SEQ ID NO: 1, SEQ ID NO:18, SEQ ID NO:19, SEQ ID NO:20, SEQ ID NO:21, SEQ ID NO:23, or SEQ ID NO:22, and wherein the PS4 variant or an amylase active fragment thereof has an amino acid substitution at one or more of the following positions 31, 58, 172, 230, 362 118, 209, 249, 301, 369, 377, 404, 407 and 421.

A PS4 variant according to the present invention is an "active amylase" or is "amylase active". In other words, the PS4 variant according to the present invention has amylase activity.

The present invention presents fragments of PS4 variants which are active amylases. In other words, the fragments of PS4 variants have amylase activity.

The amylase activity of the PS4 variant or an amylase active fragment thereof according to the present invention may be determined by any suitable method, e.g. by assays known in the art or described herein.

For example, amylase activity may be determined by using suitable assays such as the assay described in Assay 1 or Example 9.

Amylase activity of the PS4 variant or an amylase active fragment thereof or a reference polypeptide may be determined by measuring the concentration of reducing maltodextrins generated upon incubation of the amylase variant, amylase active fragment thereof or reference polypeptide with wheat starch.

The principle of the assay is as follows: The bicinchoninic acid assay (BCA assay) is used to quantify the reducing maltodextrins generated upon incubation of amylase variants, amylase active fragments thereof or reference polypeptides with wheat starch. BCA Protein Assay Kits known in the art, such as PierceTM BCA Protein Determination kit may be used.

### Assay 1 - Amylase activity

1. 2% (w/w) wheat starch in 50 mM Na-Acetate, 5 mM CaCl₂, 0.001% Triton, pH 5.5 is used as substrate.
2. 55 µL substrate is mixed with 5 µL enzyme solution and incubated at 80°C for 15 minutes.
3. After incubation, 90 µl BCA reagent (Pierce^{™} BCA Protein Determination Kit, Pierce Chemical, cat number 23225, prepared as described by manufacture) is mixed with 10 µl enzyme-starch suspension and incubated at 95°C for 10 minutes.
4. Then 70 µl of the mixture is transferred to a microtiter plate containing 80 µL H₂O, mixed and the absorbance measured at 562 nm.

In one embodiment, the present invention provides an amylase active fragment of a PS4 variant.

Preferably, the PS4 variant or amylase active fragment according to the present invention is a non-maltogenic variant.

Maltogenic amylases (EC 3.2.1.133, glucan-1,4-alpha-maltohydrolase) hydrolyze starch and remove successive α-maltose from the non-reducing end of the polysaccharide chain.

Preferably, the PS4 variant or amylase active fragment according to the present invention is an exoamylase variant.

Preferably, the PS4 variant or amylase active fragment according to the present invention is a non-maltogenic exoamylase variant.

Enzymatically active "fragments" (such as amylase active fragments) of polypeptides comprise fewer amino acids than the full-length protein but exhibit at least one activity (such as amylase activity) of the corresponding full-length protein. For example, amylase active fragments may comprise a domain or motif with at least one activity of the PS4 amylase variant, such as a catalytic domain. An amylase active fragment of a PS4 variant of the disclosure may be a polypeptide which is for example about 50, 100, 150, 200, 250, 300, 350, 400 amino acids in length. An amylase active fragment of a PS4 variant of the disclosure may be a polypeptide which is shorter than SEQ ID NO: 1, for example at least one, at least two, at least 3 at least 4 amino acids shorter than SEQ ID NO:1 or at least about 5, 10, 15, 20, 25, 30, 40, 50, 70, 75, 80, 85, 90, 95 or 100 amino acids shorter than SEQ ID NO:1.

The PS4 variant or an amylase active fragment thereof according to the present invention comprises an amino acid substitution at one or more of the following positions 31, 58, 172, 230, 362, 118, 209, 249, 301, 369, 377, 404, 407, and 421, said positions being defined with reference to SEQ ID NO: 1.

Positions which may be substituted to achieve a variant of the disclosure are defined with reference to SEQ ID NO:1. The variant or amylase active fragment thereof has an amino acid sequence which, when aligned with the amino acid sequence as set out in SEQ ID NO:1, comprises at least one substitution of an amino acid residue at a position corresponding to any of the positions 31, 58, 172, 230, 362, 118, 209, 249, 301, 369, 377, 404, 407, and 421 with reference to SEQ ID NO:1.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 1 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 2 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 3 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 4 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 5 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 6 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 7 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 9 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 10 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 11 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 12 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 13 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 14 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 15 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 16 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 17 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 18 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 19 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 20 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 21 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 22 and comprises an amino acid substitution at one or more of the following positions 31, 58, 172, 230, 362, 118, 209, 249, 301, 369, 377, 404, 407, and 421.

In one aspect, the PS4 variant or amylase active fragment thereof has at least 70% (such as at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%) sequence identity to the amino acid sequence of SEQ ID NO: 23 and comprises an amino acid substitution at one or more of the following positions 31, 58, 172, 230, 362, 118, 209, 249, 301, 369, 377, 404, 407, and 421.

In one embodiment, the present invention provides a PS4 variant or amylase active fragment thereof comprising a sequence having at least 70% sequence identity to the amino acid sequence of SEQ ID NO: 1, SEQ ID NO:18, SEQ ID NO:19, SEQ ID NO:20, SEQ ID NO:21, SEQ ID NO:23, or SEQ ID NO:22 and wherein the PS4 variant or an amylase active fragment thereof comprises at least one substitution of an amino acid residue at a position corresponding to any of the positions 31, 58, 172, 230, 362, 118, 209, 249, 301, 369, 377, 404, 407, and 421 with reference to SEQ ID NO:1, said positions being defined with reference to SEQ ID NO:1.

Preferably, the PS4 variant has at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence of SEQ ID NO: 1, SEQ ID NO:18, SEQ ID NO:19, SEQ ID NO:20, SEQ ID NO:21, SEQ ID NO:23 or SEQ ID NO:22.

Preferably, the PS4 variant or an amylase active fragment thereof has one or more amino acid substitution corresponding to 31V, 58P, 172R, 230S, 362P, 118E, 209N, 249S, 301P, 369F, 377Q, 404L, 407F or 421S.

More preferably, the PS4 variant or an amylase active fragment thereof has one or more of the following amino acid substitutions 68S, 145Q, 155P, 220M, 3V, 1101, 169H, 179V, 188E, 188R, 205Q, 313A, 313W, 367T, 399E, 422Y, 1101, 179N, 188E or 313W.

Still more preferably, the PS4 variant or an amylase active fragment thereof has one or more of the following substitutions 34Q, 145D, 179R, 200G, 223W, 169R, 179N or 420G.

Yet more preferably, the PS4 variant or an amylase active fragment thereof has an amino acid sequence that has at least 70% sequence identity with SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16 or SEQ ID NO:17.

More preferably, the PS4 variant or an amylase active fragment thereof has an amino acid sequence that has at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, and 99% sequence identity with SEQ ID NO:2, SEQ ID NO:3, SEQ ID NO:4, SEQ ID NO:5, SEQ ID NO:6, SEQ ID NO:7, SEQ ID NO:8. SEQ ID NO:9, SEQ ID NO:10, SEQ ID NO:11, SEQ ID NO:12, SEQ ID NO:13, SEQ ID NO:14, SEQ ID NO:15, SEQ ID NO:16 or SEQ ID NO:17.

In the most preferred embodiments, the PS4 variant or an amylase active fragment thereof has the amino acid sequence of with SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16 or SEQ ID NO:17.

Preferably, the PS4 variant or an amylase active fragment thereof has a melting temperature at least about 0.5°C, 1, 2°C, 3°C, 4°C, 5°C, 6°C or 7°C greater than the melting temperature of SEQ ID NO:18.
The melting temperature of the (PS4 variant or an amylase active fragment thereof according to the present invention may be determined by any suitable method, e.g. by assays known in the art or described herein.

Melting temperature may be determined by using suitable techniques such as the Nano differential scanning fluorimetry (nanoDSF) technique, for example the technique described in Example 10.

In one embodiment, the variant or an amylase active fragment thereof according to the present invention demonstrates a melting temperature above the melting temperature of SEQ ID NO 18.

Preferably, the PS4 variant or an amylase active fragment thereof has an exo to endo activity ratio of at least about 50%, 75%, 100%, 200%, 300%, 400%, 500%, 600%, 700% or 800% of the exo to endo activity ratio of SEQ ID NO:18.

The "EXO activity" of the PS4 variant or an amylase active fragment thereof according to the present invention may be determined by any suitable method, e.g. by assays known in the art or described herein.

EXO activity may be determined by using suitable assays such as the Betamyl-G6 assay, for example the assay described in Example 7.

The "ENDO activity" of the PS4 variant or an amylase active fragment thereof according to the present invention may be determined by any suitable method, e.g. by assays known in the art or described herein.

ENDO activity may be determined by using suitable assays such as the Ceralpha assay, for example the assay described in Example 8.

In another aspect of the present invention, use of a PS4 variant or an amylase active fragment thereof as set forth above as a food or feed additive is presented.

In another aspect of the present invention, a process is presented for treating starch by contacting starch with a PS4 variant or an amylase active fragment thereof as described above and allowing the PS4 variant or an amylase active fragment thereof to generate from the starch one or more linear products.

In another aspect of the present invention, the use of a PS4 variant or an amylase active fragment thereof as described above in preparing a food or feed product is presented.

In another aspect of the present invention, a process for preparing a food or feed product is presented having the step of admixing a PS4 variant or an amylase active fragment thereof as described above with a food or feed ingredient.

Preferably, the food product in the use or process is a dough or a dough product. More preferably, the dough product is a processed dough product.

Preferably, the food product in the use or process is a bakery product.

In one embodiment, the PS4 variant or an amylase active fragment thereof according to the present invention demonstrates an improved performance index (PI) value as described herein. In one embodiment, the PS4 variant or an amylase active fragment thereof according to the present invention demonstrates an improved baking performance index (PI) value as described herein. Suitably, the PS4 variant or an amylase active fragment thereof according to the present invention may demonstrate an improved performance index (PI) value of at least one of the following: PI exo activity; PI exo to endo activity ratio; PI activity on wheat starch; PI softness and/or PI resilience. A PI value above one is considered as desirable in terms of exo activity, exo to endo activity ratio, activity on wheat starch, softness and resilience.

Suitably, the PS4 variant or an amylase active fragment thereof according to the present invention may demonstrate a PI value of above 1.0 for at least one of the following: PI exo activity; PI exo to endo activity ratio; PI activity on wheat starch; PI softness and/or PI resilience. The PI value for softness may be calculated at day 7 or at day 21. The PI value for resilience may be calculated at day 7 or at day 21.

PI exo activity; PI exo to endo activity ratio; PI activity on wheat starch; PI softness and PI resilience Performance index may be calculated as described in Example 13.

Briefly, the PI for EXO activity, ENDO activity and wheat starch may be calculated at identical protein concentrations as the OD of a given variant divided by the OD of SEQ ID NO:18. EXO/ENDO ratio may be calculated as the OD of the EXO activity assay divided by the OD of the ENDO activity assay. PI for EXO to ENDO ratio may be calculated as the EXO to ENDO ratio of a given variant divided by the EXO to ENDO ratio of SEQ ID NO:18. The PI Softness may be calculated as the hardness value of SEQ ID NO:18 divided by the hardness value of the given variant.

The PI for resilience may be calculated as the resilience of a given variant divided by the resilience of SEQ ID NO: 18.

In one embodiment, the present invention provides the use of a PS4 variant or amylase active fragment thereof for improving at least one PI value as described herein, such as a baking PI value.

In one embodiment, the present invention provides a method for improving at least one PI value as described herein, such as a baking PI value in a dough, dough product, processed dough product, or a bakery product.

In one embodiment, the present invention enables the production of bakery products, such as breads, which maintain a high level of softness and resilience after 21 days of storage. Suitably, the PSV variants or amylase active fragments thereof according to the present invention enable the production of bakery products, such as breads, which demonstrate a higher level of softness and resilience after 21 days of storage when compared with comparable bakery products, such as breads, produced with SEQ ID NO:18 rather than the PSV variant or amylase active fragment according to the present invention. According to another aspect of the present invention, a process for making a bakery product is presented having the steps of (a) providing a starch medium; (b) adding to the starch medium a PS4 variant or an amylase active fragment thereof as described above; and (c) applying heat to the starch medium during or after step (b) to produce a bakery product.

In another aspect of the present invention a food product, feed product, dough product or a bakery product obtained by a use or process as described above is presented.
In another aspect of the present invention, a process is presented for treating starch by contacting starch with a PS4 variant or an amylase active fragment thereof as described above and allowing the PS4 variant or an amylase active fragment thereof to generate from the starch one or more linear products.

In another aspect of the present invention, the use of a PS4 variant or an amylase active fragment thereof as described above in preparing a food or feed product is presented.

In another aspect of the present invention, a process for preparing a food or feed product is presented having the step of admixing a PS4 variant or an amylase active fragment thereof as described above with a food or feed ingredient.

Preferably, the food product in the use or process is a dough or a dough product. More preferably, the dough product is a processed dough product.

Preferably, the food product in the use or process is a bakery product.

According to another aspect of the present invention, a process for making a bakery product is presented having the steps of (a) providing a starch medium; (b) adding to the starch medium a PS4 variant or an amylase active fragment thereof as described above; and (c) applying heat to the starch medium during or after step (b) to produce a bakery product.

In another aspect of the present invention a food product, feed product, dough product or a bakery product obtained by a use or process as described above is presented.

According to another aspect of the present invention, a method of making a saccharide syrup is presented having the step of adding a PS4 variant or an amylase active fragment thereof as described above to a granular starch liquefact to form a saccharide syrup.

In another aspect of the present invention, a dough is presented having a PS4 variant or an amylase active fragment thereof as set forth above. Preferably, the dough has at least one additional enzyme useful for improving dough and/or a baked product made therefrom. Preferably, the additional enzyme is one or more of an amylase wherein the amylase is different than the PS4 variant or an amylase active fragment thereof as set forth above, cyclodextrin glucanotransferase, peptidase, transglutaminase, lipase, galactolipase, phospholipase, cellulase, hemicellulase, protease, protein disulfide isomerase, glycosyltransferase, peroxidase, lipoxygenase, laccase, and oxidase. More preferably, the additional enzyme is an amylase. Still more preferably, the amylase is an exoamylase. In yet more preferred embodiments, the exoamylase is a maltogenic amylase. Most preferably, the maltogenic amylase is a polypeptide according to SEQ ID NO:24. In other preferred embodiments, the exoamylase is a non-maltogenic amylase or a phospholipase or a galactolipase.

In another aspect, a method is presented of preparing a baked product having the step of baking a dough as described above. Another aspect is such a baked product.

In another aspect of the present invention, a method is presented for making a dough having the step of admixing a dough component selected from the group consisting of flour, salt, water, sugar, fat, lecithin, oil and yeast with a PS4 variant as described above. Preferably, the additional enzyme is one or more of an amylase wherein the amylase is different than the PS4 variant or an amylase active fragment thereof as set forth above, cyclodextrin glucanotransferase, peptidase, transglutaminase, lipase, galactolipase, phospholipase, cellulase, hemicellulase, protease, protein disulfide isomerase, glycosyltransferase, peroxidase, lipoxygenase, laccase, and oxidase. More preferably, the additional enzyme is an amylase. Still more preferably, the amylase is an exoamylase. In yet more preferred embodiments, the exoamylase is a maltogenic amylase. Most preferably, the maltogenic amylase is a polypeptide according to SEQ ID NO:24. In other preferred embodiments, the exoamylase is a non-maltogenic amylase or a phospholipase or a galactolipase.

In another aspect a nucleic acid is presented which is capable of encoding a PS4 variant or an amylase active fragment thereof as described above. Another aspect is an expression vector having the nucleic acid. Yet another aspect is a host cell having the expression vector. Preferably, the host cell is a bacterial, fungal or yeast cell.

Various preferred features and embodiments of the present invention will now be described with reference to the following numbered paragraphs.
1. A *Pseudomonas saccharophila* amylase (PS4) variant or an amylase active fragment thereof, wherein the variant comprises a sequence having at least 70% sequence identity to the amino acid sequence of SEQ ID NO: 1, SEQ ID NO:18, SEQ ID NO:19, SEQ ID NO:20, SEQ ID NO:21, SEQ ID NO:23, or SEQ ID NO:22, and wherein the PS4 variant comprises an amino acid substitution at one or more of the following positions 31, 58, 172, 230, 362, 118, 209, 249, 301, 369, 377, 404, 407, and 421.
2. The PS4 variant or an amylase active fragment thereof of paragraph 1 having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, and 99% sequence identity to the amino acid sequence of SEQ ID NO: 1, SEQ ID NO:18, SEQ ID NO:19, SEQ ID NO:20, SEQ ID NO:21, SEQ ID NO:23, or SEQ ID NO:22.
3. The PS4 variant or an amylase active fragment thereof of paragraph 1 or 2 wherein the amino acid substitution is one or more of the following 31V, 58P, 172R, 230S, 362P, 118E, 209N, 249S, 301P, 369F, 377Q, 404L, 407F or 421S.
4. The PS4 variant or an amylase active fragment thereof of any of paragraphs 1 to 3 further comprising one or more of the following amino acid substitutions 68S, 145Q, 155P, 220M, 3V, 1101, 169H, 179V, 188E, 188R, 205Q, 313A, 313W, 367T, 399E, 422Y, 1101, 179N, 188E or 313W.
5. The PS4 variant or an amylase active fragment thereof of any of the preceding paragraphs further comprising 34Q, 145D, 179R, 200G, 223W, 169R, 179N or 420G.
6. The PS4 variant or an amylase active fragment thereof of any of the preceding paragraphs comprising an amino acid sequence that has at least 70% sequence identity with an amino acid sequence of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, or SEQ ID NO:17.
7. The PS4 variant or an amylase active fragment thereof of paragraph 6 comprising an amino acid sequence that has at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, and 99% sequence identity with the amino acid sequence SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO: 15, SEQ ID NO:16, or SEQ ID NO:17.
8. The PS4 variant or an amylase active fragment thereof of paragraph 7 comprising the amino acid sequence of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, or SEQ ID NO:17.
9. The PS4 variant or an amylase active fragment thereof of any of the preceding paragraphs having a melting temperature at least about 0.5°C, 1, 2°C, 3°C, 4°C, 5°C, 6°C or 7°C greater than the melting temperature of SEQ ID NO:18.
10. The PS4 variant or an amylase active fragment thereof of any of the preceding paragraphs having an exo to endo activity ratio of at least about 50%, 75%, 100%, 200%, 300%, 400%, 500%, 600%, 700% or 800% of the exo to endo ratio of SEQ ID NO:18.
11. Use of a PS4 variant or an amylase active fragment thereof according to any preceding paragraph as a food or feed additive.
12. A process for treating starch comprising contacting starch with a PS4 variant or an amylase active fragment thereof according to any of paragraphs 1 to 10 and allowing the polypeptide to generate from the starch one or more linear products.
13. Use of a PS4 variant or an amylase active fragment thereof according to any of paragraphs 1 to 10 in preparing a food or feed product.
14. A process for preparing a food or feed product comprising admixing a PS4 variant or an amylase active fragment thereof according to any of paragraphs 1 to 10 with a food or feed ingredient.
15. Use according to paragraph 11 or paragraph 13, or a process according to paragraph 14, wherein the food product is a dough or a dough product, preferably a processed dough product.
16. Use according to paragraph 11 or paragraph 13, or a process according to paragraph 14, wherein the food product is a bakery product.
17. A process for making a bakery product comprising: (a) providing a starch medium; (b) adding to the starch medium a PS4 variant or an amylase active fragment thereof according to any of paragraph s 1 to 10; and (c) applying heat to the starch medium during or after step (b) to produce a bakery product.
18. A food product, feed product, dough product or a bakery product obtained by a use or process according to any of paragraphs 11 to 17.
19. A method of making a saccharide syrup, comprising adding a PS4 variant or an amylase active fragment thereof according to any of paragraphs 1 to 10 to a granular starch liquefact to form a saccharide syrup.
20. A dough comprising a PS4 variant or an amylase active fragment thereof according to any of paragraphs 1 to 10.
21. A dough according to paragraph 20 further comprising at least one additional enzyme useful for improving dough and/or a baked product made therefrom.
22. A dough according to paragraph 21 wherein the additional enzyme is one or more of an amylase wherein the amylase is different than the PS4 variant or an amylase active fragment thereof as set forth in paragraph s 1 to 10, cyclodextrin glucanotransferase, peptidase, transglutaminase, lipase, galactolipase, phospholipase, cellulase, hemicellulase, protease, protein disulphide isomerase, glycosyltransferase, peroxidase, lipoxygenase, laccase, and oxidase.
23. A dough according to paragraph 22 wherein the additional enzyme is an amylase.
24. A dough according to paragraph 23 wherein the amylase is an exoamylase
25. A dough according to paragraph 24 wherein said exoamylase is a maltogenic amylase.
26. A dough according to paragraph 25 wherein the maltogenic amylase is a polypeptide according to SEQ ID NO:24.
27. A dough according to paragraph 23 wherein said exoamylase is a non-maltogenic amylase.
28. A dough according to paragraph 22 wherein said additional enzyme is a phospholipase or a galactolipase.
29. A method of preparing a baked product comprising baking a dough according to any of paragraphs 20 to 28.
30. A baked product according to paragraph 29.
31. A method of making a dough, said method comprising admixing a dough component selected from the group consisting of flour, salt, water, sugar, fat, lecithin, oil and yeast with a PS4 variant or an amylase active fragment thereof according to any of paragraph s 1 to 10.
32. A method of making a dough according to paragraph 31 further comprising adding at least one additional enzyme useful for improving dough and/or a baked product made therefrom.
33. A method according to paragraph 32 wherein the additional enzyme is selected from the group consisting of amylase wherein the amylase is different than the PS4 variant or an amylase active fragment thereof as set forth in claims 1 to 10, cyclodextrin glucanotransferase, peptidase, transglutaminase, lipase, galactolipase, phospholipase, cellulase, hemicellulase, protease, protein disulphide isomerase, glycosyltransferase, peroxidase, lipoxygenase, laccase, and oxidase.
34. A method according to paragraph 33 wherein the additional enzyme is an amylase.
35. A method according to paragraph 34 wherein said amylase is an exoamylase.
36. A method according to paragraph 35 wherein said exoamylase is a maltogenic amylase.
37. A method according to paragraph 36 wherein the maltogenic amylase is a polypeptide according to SEQ ID NO:24.
38. A method according to paragraph 35 wherein said exoamylase is a non-maltogenic amylase.
39. A method according to paragraph 33 wherein said additional enzyme is a phospholipase or a galactolipase.
40. A nucleic acid capable of encoding a PS4 variant according to any of paragraphs 1 to 10.
41. An expression vector comprising a nucleic acid according to paragraph 40.
42. A host cell comprising an expression vector according to paragraph 1.
43. A host cell according to paragraph 42 which is a bacterial, fungal or yeast cell.

The invention will now be further described by way of Examples, which are meant to serve to assist one of ordinary skill in the art in carrying out the invention and are not intended in any way to limit the scope of the invention.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

### EXAMPLE 1

### Generation of amylase variant library clones

Generation of variant libraries were performed using molecular techniques known in the art. Backbone DNA sequences encoding mature amylase proteins, carrying all mutations to be incorporated in the final variants were ordered synthetically as gene fragments from Twist Biosciences (San Francisco, California, USA). These gene fragments were amplified in order to incorporate uracil in the sequences. These sequences were fragmented by methods know in the art prior to assembly of all the sequences by PCR. The resulting mature amylase encoding DNA sequence fragments were in-frame fused to the *Bacillus licheniformis amyL* signal peptide DNA sequence at the 5' end, and the *Bacillus licheniformis amyL* transcription terminator sequence at the 3' end. In addition, a constitutive promoter was fused to the 5' end of the signal peptide sequence, and an antibiotic resistance marker DNA cassette together with 3' and 5'-end flanking DNA regions homologous to *Bacillus licheniformis* were added to the expression cassettes as well, enabling homologous recombination and selective growth of *Bacillus licheniformis* transformants. These DNA cassettes were transformed into *Bacillus licheniformis* cells using methods known in the art. Transformants were grown on selective agar plates and in selective liquid medium by making use of the appropriate antibiotic resistance selection. For all individual library clones, DNA sequence encoding the amylase variant expression cassette was PCR amplified and analysed by Pac Bio next gen sequencing to identify the DNA sequence of the amylase variant.

### EXAMPLE 2

### Generation of individual amylase variant clones

DNA sequences encoding mature amylase proteins were ordered synthetically as gene fragments from Twist Biosciences (San Francisco, California, USA) or IDT (Integrated DNA Technologies, BVBA, Belgium). These mature amylase encoding DNA gene fragments were in-frame fused to the *Bacillus licheniformis amyL* signal peptide DNA sequence at the 5' end, and the *Bacillus licheniformis amyL* transcription terminator sequence at the 3' end. In addition, a constitutive promoter was fused to the 5' end of the signal peptide sequence, and an antibiotic resistance marker DNA cassette together with 3' and 5'-end flanking DNA regions homologous to *Bacillus licheniformis* were added to the expression cassettes as well, enabling homologous recombination and selective growth of *Bacillus licheniformis* transformants. These DNA cassettes were transformed into *Bacillus licheniformis* cells using methods known in the art. Transformants were grown on selective agar plates and in selective liquid medium by making use of the appropriate antibiotic resistance selection. For each individual clone, DNA sequence encoding the amylase variant expression cassette was PCR amplified and analysed by Sanger sequencing to identify the DNA sequence of the amylase variant.

### EXAMPLE 3

### Generation of amylase variant protein sample

For amylase protein sample generation, individual (library) clones were grown at microtiter plate scale, using an enriched MOPS based semi-defined liquid medium, and cultured in an orbital shaking incubator. Cell culture broth samples were spun down to filter supernatant samples for further usage.

### EXAMPLE 4

### Generation of amylase variant samples for testing purposes

For generation of amylase variant samples for testing purposes selected strains were grown in shake flasks or in 2 or 10 litre bioreactors.

In shake flasks an enriched MOPS based semi-defined liquid medium were used and the flasks were incubated in an orbital shaking incubator. Cell culture broth samples were spun down and supernatants sterile filtered for usage.

In bioreactors glucose limited fed batch fermentation based on semi-defined liquid medium were used. Samples were obtained after fermentation by cell separation filtration, ultra-filtration and sterile filtration.

### EXAMPLE 5

### HPLC

Protein concentration was determined applying HPLC. In a 384-well plate samples were prepared by mixing 20 µl of MTP supernatant with 80 µl of MOPS-buffer, pH 7.15, with 10 mM CaCl₂ and 0.005% Tween 80. The system used was Agilent (U)HPLC, LC 1290 infinity with hip-ALS injection system and the column used was a Zorbax 300 SB-C3 column 2.1x50 mm with 1.8 µm beadsize. 5 µL of sample was injected with a 3 second needle flush with demi-water. A flow of 1.00 ml/min was used and the gradient applied is shown in Table 4 below. Optical density (OD) was read at 220 nm. A standard curve of SEQ ID NO:18 was in each plate and provided the calibration points for the samples in each separate plate.

**Table 4: Gradient used for HPLC analysis of SEQ ID NO: 18 variants.**

| Time (min) | %A | %B |
|---|---|---|
| 0 | 95 | 5 |
| 0.2 | 95 | 5 |
| 0.6 | 65 | 35 |
| 0.8 | 65 | 35 |
| 3.5 | 40 | 60 |
| 3.6 | 5 | 95 |
| 4.0 | 5 | 95 |
| 4.1 | 95 | 5 |
| 4.4 | 95 | 5 |

Solvent A above is water + 0.1% TFA and solvent Bis acetonitrile+ 0.07% TFA.

### EXAMPLE 6

### Protein Determination by Stain Free Imager Criterion

Protein was quantified by SDS-PAGE gel and densitometry using Gel Doc^{™} EZ imaging system. Reagents used in the assay: Concentrated (2x) Laemmli Sample Buffer (Bio-Rad, Catalogue #1610737); 26-well TGX Any kDa Gel (Bio-Rad, Catalogue #5678125); protein markers "Precision Plus Protein^{™} Unstained Protein Standards, Strep-tagged recombinant, 1 ml" (Bio-Rad, Catalogue #161-0363); protein standard BSA (Thermo Scientific, Catalogue #23208) and SimplyBlue Safestain (Invitrogen, Catalogue #LC 6060. The analysis was carried out as follow: In a 96well-PCR plate 50µL diluted enzyme sample were mixed with 50 µL sample buffer containing 2.7 mg DTT*. The plate was sealed by Microseal 'B' Film from Bio-Rad and was placed into PCR machine to be heated to 70°C for 10 minutes. Then the chamber was filled with running buffer and the gel cassette inserted. Next 10 µL of each sample and standard (0.125-1.00 mg/mL SEQ ID NO:18) was loaded on the gel and 10 µL of the markers were loaded. After that the electrophoresis was run at 200 V for 34 min. Following electrophoresis the gel was then transferred to the Gel Doc^{™} EZ imaging system and the amount of target protein in the sample was determined using the Image Lab software and the calibration curve.

### EXAMPLE 7

### Betamyl assay (Exo-activity)

The betamyl-G6 assay is based on the hydrolysis of PNP-maltohexaoside, releasing the yellow coloured PNP anion under alkaline conditions, which can be quantified by UV/VIS spectrometry. Alpha-glucosidase is added as an auxiliary enzyme hydrolysing the PNP-maltopyranoside product remaining after hydrolysis by the amylase. 25 µL 12 mM 4-Nitrophenyl α-D-maltohexaoside (PNPG6) (Sigma Aldrich, cat. No. 73681), 23 U/ml α-glucosidase (Megazyme, cat. no. E-MALTS) in 50 mM Na-Acetate, 5 mM CaCl₂, 0.001% Triton, pH 5.5 was mixed with 65 µL 50 mM Na-Acetate, 5 mM CaCl₂, 0.001% Triton, pH 5.5 and 10 µL enzyme solution in a MTP. Then the plate was incubated at 30C for 15 min. Next 150 µL 4% TRIS base was added and OD 410 nm was measured.

### EXAMPLE 8

### Ceralpha assay (Endo-activity)

The Ceralpha assay is based on the hydrolysis of PNP-Phenyl-maltoheptaoside releasing the yellow coloured PNP anion under alkaline conditions which can be quantified by UV/VIS spectrometry. This substrate has a phenyl-group attached to the non-reducing end which sterically hinders exo-activity. Hence this substrate is specific for endo-amylases. Alpha-glucosidase and glucoamylase in excess are an integrated part of the Ceralpha assay kit. These auxiliary enzymes ensure complete hydrolysis releasing the PNP-anion after the initial endo hydrolysis. 25 µL Ceralpha substrate (Megazyme, cat. No. R-AMHR4, prepared according to manufactures description) was mixed with 50 µL 50 mM Na-Acetate, 5 mM CaCl₂, 0.001% Triton, pH 5.5 and 10 µL enzyme solution in a MTP. Then the plate was incubated at 30C for 15 min. Next 150 µL 4% TRIS base was added and OD 410 nm was measured.

### EXAMPLE 9

### Activity towards wheat starch

The principle behind the activity assay is to measure the concentration of reducing maltodextrins generated upon incubation of amylase variants with wheat starch. The Bicinchoninic Acid (BCA) assay is used to quantify the reducing maltodextrins. 2% (w/w) wheat starch in 50 mM Na-Acetate, 5 mM CaCl₂, 0.001% Triton, pH 5.5 was used as substrate. 55 µL substrate was mixed with 5 µL enzyme solution and incubated at 80°C for 15 min. After incubation, 90 µl BCA reagent (PierceTM BCA Protein Determination kit, Pierce Chemical, cat number 23225, prepared as described by manufacture) was mixed with 10 µl enzyme-starch suspension and incubated at 95°C for 10 min. Then 70 µl was transferred to a microtiter plate containing 80 µL H₂O, mixed and the absorbance measured at 562 nm.

### EXAMPLE 10

### Melting temperature, Tm

The unfolding temperature of proteins were measured using nanoDSF technique applying Prometheus NT.48 from Nanotemper. All samples were diluted to 0.25 mg/ml in 50 mM Na-Acetate, 5 mM CaCl₂, 0.001% Triton, pH 5.5 and a melting scan from 60°C -110°C with gradient of 1°C/min was conducted.

### EXAMPLE 11

### Baking performance evaluation

### Baking procedure

Baking trials were carried out with a standard white bread straight dough recipe for US toast. The straight dough is prepared from 2000 g of Wheat flour from Grain Craft, USA, 1220 g of tap-water, 40 g of Rapeseed Oil (Prod. No. 3520, AAK, Denmark), 160 g Sucrose, 40 g NaCl, 7 g of calcium propionate (Dupont Nutrition Bioscience, Denmark), 80 g compressed yeast (S. cerevisiae, HAGOLD HEFE, Schwarzenbach a.d. Saale, Germany) and 0,15 g ascorbic acid (Dupont Nutrition Bioscience, Denmark). The following enzymes are used beside the enzymes for anti-staling; 50ppm GRINDAMYL^{®} SUREBake 800 a hexose oxidase (Dupont Nutrition Bioscience, Denmark), 25ppm GRINDAMYL^{™} POWERBake 950 a xylanse (Dupont Nutrition Bioscience, Denmark) and 275ppm GRINDAMYL^{®} A 1000 fungal alpha-amylase as target depending on the falling number of the flour (Dupont Nutrition Bioscience, Denmark).

All ingredients were mixed subsequently 2 min. at speed 2 on a Hobart A200 spiral mixer and following 18 minutes medium speed with a dough temperature of 26-27°C. The dough was afterwards scaled into 4 dough pieces at 703 g = 5,11 cubic inch/oz dough pieces, rested for 5 minutes at ambient temperature and moulded on a cross grain moulder, Benier MS500 with the following settings: Preform: -18, Drum press.: 3, Pressure board: 4,0 cm front, 3,5 cm back, Width: 350 mm front, 320 mm back. Hereafter the dough was transferred to pans/tins and proofed for 55 min. at 43°C with 75% RH and then baked for 25 min. at 205°C in a Reed Rack Oven. The final loaf temperature was verified to be 96-98°C and after baking the breads were cooled for 60 minutes at ambient temperature before weighing and measuring of volume. The bread used for hardness and resilience measurements were packed with vacuum.

### EXAMPLE 12

### Evaluation of Hardness and Resilience.

Texture Profile Analysis (TPA) of Bread Hardness and Resilience were determined by analysing bread slices by TPA using a Texture Analyser from Stable Micro Systems, UK (SMS). Calculation of hardness and resilience was done according to preset standard supplied by Stable Micro System. The probe used was aluminum 50 mm round. Measurements were performed by cutting bread slices (4 pieces) into small 'discs' with a width of 45 mm, placing one slice/disc at a time on the Texture Analyser and then compressed with a dept of 60% (two compression). The following settings were used: Pre Test Speed: 2 mm/s, Test Speed: 2 mm/s, Post Test Speed: 10 mm/s, target mode: Strain, Trigger Force: 1.0g, Time between measurements: 2.00 sec, Count: 5, Load Cell: 5 kg, Trigger Type: Auto. The mode of compression is a modification to the one used in Standard method AACC 74-09. The sample was compressed twice in the test.
Hardness (expressed in Newton) is measured as the gram force at the top peak point during the first compression. The lower the hardness value, the softer the bread. The measured hardness value is used to calculate the PI softness value given in table 6.
Resilience is calculated as the area under the curve during withdrawal relative to the area under the curve during compression.

### EXAMPLE 13

### Data for disclosed variants.

Performance index (PI) for EXO activity, ENDO activity and Wheat starch was calculated at identical protein concentrations as the OD of a given variant divided by the OD of SEQ ID NO:18. EXO/ENDO ratio was calculated as the OD of the EXO activity assay divided by the OD of the ENDO activity assay. PI for EXO to ENDO ratio was calculated as the EXO to ENDO ratio of a given variant divided by the EXO to ENDO ratio of SEQ ID NO:18.

In contrast to the other PI values presented in table 6, where the PI is calculated as the value of the variant is divided by the value of SEQ ID NO 18, the PI Softness is calculated as the Hardness value of SEQ ID NO:18 divided by the Hardness value of the given variant. This is done to reflect that softness is the desired property and so that a PI value on softness above one for a given variant indicates that the bread made with the variant is softer (i.e. has a lower hardness) compared to a bread made with SEQ ID NO 18.
PI for Resilience was calculated as the Resilience of a given variant divided by the Resilience of SEQ ID NO:18.
A PI value above one is considered as desirable in terms of exo activity, exo to endo activity ratio, activity on wheat starch, softness and resilience. Additionally, a melting temperature (Tm) above the melting temperature of SEQ ID NO 18 is considered desirable.

Data for some of the disclosed variants is set forth below in Table 6.

**Table 6.**

| SEQ ID NO: | PI exo-activity | PI endoactivit y | PI exo to endo activit y | PI wheat starch | Tm in °C | PI Soft - ness day 7 | PI Softnes s day 21 | PI Resilienc e day 7 | PI Resilienc e day 21 |
|---|---|---|---|---|---|---|---|---|---|
| 2 | 1.4 | 0.4 | 3.6 | 2.1 | 96.0 | 1.0 | 1.1 | 1.2 | 1.3 |
| 3 | 1.5 | 0.4 | 3.5 | 3.3 | 92.1 | 1.2 | 1.0 | 1.2 | 1.4 |
| 4 | 1.0 | 0.5 | 2.1 | 1.3 | 95.2 | 1.2 | 1.1 | 1.2 | 1.4 |
| 5 | 0.7 | 0.5 | 1.4 | 0.7 | 93.0 | 1.4 | 1.3 | 1.2 | 1.2 |
| 6 | 1.0 | 0.3 | 3.2 | 0.9 | 92.4 | 1.2 | 1.2 | 1.3 | 1.4 |
| 7 | 1.4 | 0.3 | 4.1 | 1.3 | 96.0 | 1.4 | 1.4 | 1.2 | 1.1 |
| 8 | 1.2 | 0.2 | 5.6 | 1.2 | 93.2 | 1.2 | 1.4 | 1.3 | 1.4 |
| 9 | 1.1 | 0.3 | 3.8 | 1.1 | 95.3 | 1.3 | 1.6 | 1.4 | 1.4 |
| 10 | 1.0 | 0.3 | 4.0 | 1.0 | 96.4 | 1.7 | 1.6 | 1.3 | 1.4 |
| 11 | 1.3 | 1.0 | 1.3 | 2.5 | 96.1 | 1.2 | 1.0 | 1.2 | 1.2 |
| 12 | 1.4 | 0.7 | 1.9 | 2.0 | 96.4 | 1.5 | 1.2 | 1.1 | 1.1 |
| 13 | 1.1 | 0.6 | 2.0 | 1.4 | 96.4 | 1.2 | 1.1 | 1.2 | 1.3 |
| 14 | 2.4 | 1.3 | 1.9 | 4.4 | 96.1 | 1.0 | 1.0 | 1.1 | 1.2 |
| 15 | 0.9 | 1.1 | 0.8 | 1.1 | 96.4 | 1.1 | 1.1 | 1.0 | 1.1 |
| 16 | 1.7 | 0.4 | 4.5 | 0.8 | 94.6 | 1.1 | 1.0 | 1.2 | 1.4 |
| 17 | 0.9 | 0.7 | 1.3 | 0.7 | 92.0 | 1.5 | 1.0 | 1.2 | 1.2 |
| 18 | 1.0 | 1.0 | 1.0 | 1.0 | 90.7 | 1.0 | 1.0 | 1.0 | 1.0 |

Data for some of the individual substitutions in a SEQ ID NO:18 background is set forth in Table 7 below.

**Table 7.**

| Positio n | Subst itutio n | PI exo-activity | PI endo-activity | PI exo to endo activity | PI wheat starch | Delta Tm in °C |
|---|---|---|---|---|---|---|
| SEQ | | 1 | 1 | 1 | 1 | 0 |

| ID NO 18 | | | | | | |
|---|---|---|---|---|---|---|
| 3 | V | 0.99 | 1.00 | 0.99 | 1.00 | -0.43 |
| 31 | V | 0.96 | 0.99 | 0.97 | 1.09 | -0.20 |
| 34 | Q | 0.99 | 0.88 | 1.12 | 0.90 | 0.27 |
| 58 | P | 1.55 | 0.77 | 2.02 | 1.74 | 1.11 |
| 68 | S | 0.94 | 0.73 | 1.28 | 0.85 | 0.26 |
| 110 | I | 1.03 | 0.95 | 1.08 | 1.01 | 0.07 |
| 118 | E | 0.99 | 0.66 | 1.49 | 0.82 | -3.81 |
| 145 | D | 0.92 | 1.01 | 0.91 | 1.11 | 3.10 |
| 145 | Q | 0.91 | 0.99 | 0.92 | 1.09 | 2.13 |
| 155 | P | 0.79 | 1.32 | 0.60 | 1.04 | 2.63 |
| 169 | H | 0.97 | 0.98 | 0.99 | 1.01 | 0.64 |
| 169 | R | 1.00 | 0.98 | 1.03 | 1.04 | 1.30 |
| 172 | R | 0.97 | 0.96 | 1.01 | 1.01 | 0.91 |
| 179 | N | 1.12 | 1.04 | 1.07 | 1.11 | 0.51 |
| 179 | R | 1.11 | 1.05 | 1.05 | 1.16 | 0.77 |
| 179 | V | 1.12 | 1.06 | 1.06 | 1.10 | 0.59 |
| 188 | E | 0.97 | 0.95 | 1.02 | 0.98 | 0.37 |
| 188 | R | 1.00 | 0.98 | 1.03 | 1.03 | 0.54 |
| 200 | G | 1.28 | 1.21 | 1.06 | 1.47 | 0.29 |
| 205 | Q | 1.01 | 1.03 | 0.99 | 1.02 | -1.25 |
| 209 | N | 1.00 | 0.99 | 1.01 | 1.06 | -1.09 |
| 220 | M | 1.32 | 1.02 | 1.29 | 1.42 | 0.08 |
| 223 | w | 0.92 | 0.57 | 1.63 | 0.53 | -0.12 |
| 230 | S | 0.98 | 1.02 | 0.97 | 0.97 | 0.77 |
| 249 | S | 0.97 | 0.96 | 1.00 | 1.01 | 0.48 |
| 301 | P | 0.93 | 0.93 | 1.01 | 0.89 | 0.44 |
| 313 | A | 0.88 | 0.96 | 0.92 | 0.93 | 0.25 |
| 313 | w | 0.89 | 0.92 | 0.97 | 1.01 | 0.38 |
| 362 | P | 0.86 | 0.86 | 0.99 | 0.89 | 0.52 |
| 367 | T | 0.96 | 1.00 | 0.96 | 0.98 | 0.39 |
| 369 | F | 0.93 | 0.93 | 0.99 | 0.95 | 0.50 |
| 377 | Q | 0.97 | 1.02 | 0.95 | 0.95 | -0.18 |
| 399 | E | 1.04 | 1.06 | 0.98 | 1.01 | 0.34 |
| 404 | L | 1.08 | 1.04 | 1.04 | 1.10 | -0.51 |
| 407 | F | 1.27 | 1.26 | 1.00 | 1.32 | -0.57 |
| 420 | G | 0.92 | 0.94 | 0.99 | 0.94 | 0.43 |
| 421 | S | 0.98 | 1.02 | 0.96 | 1.04 | 0.60 |
| 422 | Y | 0.94 | 0.96 | 0.98 | 0.99 | 0.41 |

## Claims

1. A *Pseudomonas saccharophila* amylase (PS4) variant or an amylase active fragment thereof, wherein the variant comprises a sequence having at least 70% sequence identity to the amino acid sequence of SEQ ID NO: 1, SEQ ID NO:18, SEQ ID NO:19, SEQ ID NO:20, SEQ ID NO:21, SEQ ID NO:23, or SEQ ID NO:22, and wherein the PS4 variant comprises an amino acid substitution at one or more of the following positions 31, 58, 172, 230, 362, 118, 209, 249, 301, 369, 377, 404, 407, and 421.

2. The PS4 variant or an amylase active fragment thereof of claim 1:
a) having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, and 99% sequence identity to the amino acid sequence of SEQ ID NO: 1, SEQ ID NO:18, SEQ ID NO:19, SEQ ID N0:20, SEQ ID NO:21, SEQ ID NO:23, or SEQ ID NO:22; and/or
b) wherein the amino acid substitution is one or more of the following 31V, 58P, 172R, 230S, 362P, 118E, 209N, 249S, 301P, 369F, 377Q, 404L, 407F or 421S; and/or
c) further comprising one or more of the following amino acid substitutions 68S, 145Q, 155P, 220M, 3V, 1101, 169H, 179V, 188E, 188R, 205Q, 313A, 313W, 367T, 399E, 422Y, 1101, 179N, 188E or 313W; and/or
d) further comprising 34Q, 145D, 179R, 200G, 223W, 169R, 179N or 420G; and/or
e) comprising an amino acid sequence that has at least 70% sequence identity with an amino acid sequence of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, or SEQ ID NO:17, preferably
comprising an amino acid sequence that has at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, and 99% sequence identity with the amino acid sequence SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, or SEQ ID NO:17, preferably comprising the amino acid sequence of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, or SEQ ID NO:17; and/or
f) having a melting temperature at least about 0.5°C, 1, 2°C, 3°C, 4°C, 5°C, 6°C or 7°C greater than the melting temperature of SEQ ID NO:18; and/or
g) having an exo to endo activity ratio of at least about 50%, 75%, 100%, 200%, 300%, 400%, 500%, 600%, 700% or 800% of the exo to endo ratio of SEQ ID NO:18.

3. Use of a PS4 variant or an amylase active fragment thereof according to any preceding claim as a food or feed additive or in preparing a food or feed product.

4. A process for treating starch comprising contacting starch with a PS4 variant or an amylase active fragment thereof according claim 1 or claim 2 and allowing the polypeptide to generate from the starch one or more linear products.

5. A process for preparing a food or feed product comprising admixing a PS4 variant or an amylase active fragment thereof according to claim 1 or claim 2 with a food or feed ingredient.

6. Use according to claim 3, or a process according to claim 4, wherein the food product is:
a) a dough or a dough product, preferably a processed dough product; or
b) a bakery product.

7. A process for making a bakery product comprising: (a) providing a starch medium; (b) adding to the starch medium a PS4 variant or an amylase active fragment thereof according to any of claims 1 to 10; and (c) applying heat to the starch medium during or after step (b) to produce a bakery product.

8. A food product, feed product, dough product or a bakery product obtained by a use or process according to any of claims 3 to 7.

9. A method of making a saccharide syrup, comprising adding a PS4 variant or an amylase active fragment thereof according to claim 1 or claim 2 to a granular starch liquefact to form a saccharide syrup.

10. A dough comprising a PS4 variant or an amylase active fragment thereof according to claim 1 or claim 2, preferably further comprising at least one additional enzyme useful for improving dough and/or a baked product made therefrom, preferably wherein the additional enzyme is one or more of an amylase wherein the amylase is different than the PS4 variant as set forth in claim 1 or claim 2, cyclodextrin glucanotransferase, peptidase, transglutaminase, lipase, galactolipase, phospholipase, cellulase, hemicellulase, protease, protein disulphide isomerase, glycosyltransferase, peroxidase, lipoxygenase, laccase, and oxidase, preferably wherein the additional enzyme is:
i) an amylase, preferably wherein said amylase is an exoamylase, preferably wherein said exoamylase is:
a) a maltogenic amylase, preferably wherein the maltogenic amylase is a polypeptide according to SEQ ID NO:24; or
b) a non-maltogenic amylase; or
ii) a phospholipase or a galactolipase.

11. A method of preparing a baked product comprising baking a dough according to claim 10.

12. A baked product according to claim 11.

13. A method of making a dough, said method comprising admixing a dough component selected from the group consisting of flour, salt, water, sugar, fat, lecithin, oil and yeast with a PS4 variant or an amylase active fragment thereof according to claim 1 or claim 2, preferably further comprising adding at least one additional enzyme useful for improving dough and/or a baked product made therefrom, preferably wherein the additional enzyme is selected from the group consisting of amylase wherein the amylase is different than the PS4 variant or an amylase active fragment thereof as set forth in claims 1 to 10, cyclodextrin glucanotransferase, peptidase, transglutaminase, lipase, galactolipase, phospholipase, cellulase, hemicellulase, protease, protein disulphide isomerase, glycosyltransferase, peroxidase, lipoxygenase, laccase, and oxidase;
preferably wherein the additional enzyme is:
i) an amylase, preferably wherein said amylase is an exoamylase, preferably wherein said exoamylase is:
a) a maltogenic amylase, preferably wherein the maltogenic amylase is a polypeptide according to SEQ ID NO:24; or
b) a non-maltogenic amylase; or
ii) a phospholipase or a galactolipase.

14. A nucleic acid capable of encoding a PS4 variant according to claim 1 or claim 2.

15. An expression vector comprising a nucleic acid according to claim 14 or a host cell comprising such an expression vector, preferably a bacterial, fungal or yeast cell.
